# NEW EUROPEAN PATENT SPECIFICATION

(11) **EP 0 285 682 B2**
(45) Date of publication and mention of the opposition decision: **06.08.1997**
(45) Mention of the grant of the patent: 19.06.1991
(21) Application number: 87105082.9
(22) Date of filing: 06.04.1987
(51) Int. Cl.: A61K 9/16, A61K 47/00, A61K 31/07

(54) **Process for the manufacture of vitamin preparations**
Verfahren zur Herstellung von Vitaminpräparaten
Procédé de fabrication de préparations de vitamines

(43) Date of publication of application: 12.10.1988
(73) Proprietor: F. HOFFMANN-LA ROCHE AG, 4002 Basel (CH)
(72) Inventor: Scialpi, Leonard Joseph, Andover, N.J. 07821 (US)
(74) Representative: VOSSIUS & PARTNER

(56) References cited:
- FR-A- 1 287 716
- GB-A- 993 138
- US-A- 2 756 177
- US-A- 3 749 799
- Kläui et al. in "Fat soluble vitamins, Editor R.A. Morton, Intern. Encyclopedia of food and nutrition, vol. 9, Pergamon Press 1970, pp. 128-132
- Ullmanns Encyklopädie der techn. Chemie, 4. Aufl. 1983, Band 23, pp. 629-630

## Description

The present invention is concerned with a novel process for the manufacture of preparations containing fat-soluble vitamins and/or carotenoids, as well as with the preparations so obtained.

In the prior art there are already known fat-soluble vitamin powder compositions which are useful for administration as such and also for the formulation of pharmaceutical dosage forms, e.g., tablets, capsules, powders, and the like, and for the preparation of animal feeds. U.S. patent No. 2,756,177 discloses a process for preparing dry, free-flowing powders by forming an emulsion containing a vitamin-active material, water, gelatin, and/or gum acacia and a sugar, converting the emulsion to droplets, collecting the individual droplets in a mass of starchy powder in such a manner that the vitamin-active particles formed from the droplets are kept separated from each other until their particulate form is established, and separating the vitamin-active particles from the starchy collecting powder. The vitamin-containing powder prepared according to the above process is water soluble and exhibits satisfactory stability properties for most uses. However, the material does have problems withstanding the stresses of pelletizing when used for the fortification of animal feeds. The vitamin containing material tends to lose its physical integrity under the temperature, moisture and pressure conditions of the feed pelleting process and results in a loss of the physical integrity of the product thereby compromising the stability of the fat-soluble vitamin.

It is an important object of the invention to provide a vitamin or a carotenoid containing preparation in the form of beadlets characterized by high stability and potency. A further object is to provide beadlets which will withstand the temperature, moisture and pressure of the feed pelleting process without losing their physical integrity. A still further object is to provide preparations which are water insoluble, maintain bioavailability, and exhibit superior stability when pelletized.

All these objectives have been achieved according to the process of the present invention.

This process for the manufacture of preparations in beadlet form containing one or more fat-soluble vitamins and/or one or more carotenoids comprises the steps of forming an emulsion containing the active material, water, gelatin and a sugar, converting the emulsion to droplets, collecting the droplets in a mass of starchy powder in such a manner that the droplets are kept separated from each other until their particulate form is permanently etablished and separating the obtained particles from the starchy collecting powder, and is characterized in that these particles are heat treated at a temperature of from about 90°C to about 180°C.

In accordanced with the process of the invention, the heat treatment step insolubilizes the gelatin matrix of the beadlets by a reaction between the carbonyl group of the sugar with the free amino moieties of the gelatin molecule. The resulting beadlets are water-insoluble and exhibit increased stability to the stresses of feed pelleting. The crosslinking process utilizes the ingredients employed in making the beadlet and does not require addition of a crosslinking reagent or additive to the composition.

Fat-soluble vitamin-active materials which can be used in the practice of this invention are vitamin-bearing oils, provitamins, and pure or substantially pure vitamins, both natural and synthetic, or chemical derivatives thereof and mixtures thereof. Of particular interest is the preparation of beadlets containing vitamin A-active materials, more particularly, viatamin A acetate or vitamin A palmitate, but it is also contemplated to encompass beadlets containing any fat-soluble vitamin-active material, for example, vitamins A, D, E, or K, a carotenoid such as β-carotene, astaxanthin, canthaxanthin, and the like, or mixtures of such materials.

In addition, this process is applicable to the preparation of fat soluble drugs. The final product in this case would not be soluble in the stomach but is soluble in intestinal juice.

The first step in the process of the invention comprises emulsifying the active material with water, gelatin and a sugar.

Any gelatin which has a bloom in the range of practically zero to about 300 can be employed in the practice of the present invention. Both Type A and Type B gelatin can be employed.

Among the sugars used in forming the emulsions employed herein are fructose, glucose, lactose, maltose, and invert sugar (mixture of glucose and fructose). In addition, high fructose corn syrups (mixtures of fructose and dextrose) can also be employed in the practice of the invention.

Small quantities of other ingredients including antioxidants, such as, butylated hydroxy anisole (BHA), butylated hydroxy toluene (BHT), ethoxyquin (6-ethoxy-1,2-dihydro-2,2,4-trimethyl-quinoline), and the like, humectants, such as glycerin, sorbitol, polyethylene glycols, propylene glycol, and the like, emulsifiers, such as lecithin, extenders and solubilizers and coloring agents, can also be incorporated in the emulsions of this invention.

The preparation of the vitamin and/or carotenoid containing emulsion can be effected by methods which will be apparent to those skilled in the art. As an example of a method which was found satisfactory, the following can be mentioned: the gelatin is dissolved in water with the aid of moderate heating and the active substance is then dispersed or emulsified in the solution of the gelatin. The sugar, as well as any ingredients, can be introduced into the mixture either before or after adding the active material. The mixture is agitated until all dispersoids are uniformly distributed, if necessary, by passing the mixture through a homogenizer.

The starchy powder used in the process to collect the droplets of emulsion can consist entirely of a starch and/or a starch chemically modified so as to impart to it in greater degree those characteristics found to be desirable in the collecting powder, as recited hereinbelow. The collecting powder can also contain, in addition to the starch and/or modified starch, minor amounts of lubricants and other modifiers, such as talc, silicic acid, flours, hydrogenated fats and metal salts of higher fatty acids, for example, calcium stearate. The collecting powder should possess the following characteristics: it should be substantially insoluble in cold water and, moreover, should be resistant to wetting by water; it should have an appreciable capacity to absorb and/or adsorb water; and it should be free-flowing. An important characteristic of the collecting powder is that its moisture content must be below about 10 percent. The desired moisture content can be easily attained by drying the commercially available starches or chemically modified starches.

A preferred collecting powder consists substantially entirely of a starch modified to contain hydrophobic groups, so as to possess the properties of free-flow and resistance to water-wetting to a higher degree than unmodified starch. Starch derivatives of this type, more particularly starch esters, are disclosed e.g. in United States patent No. 2,613,206. A free-flowing starch ester, resistant to water-wetting, available commercially under the designation "Dry-Flo"® and distributed by National Starch products, Inc., New York, New York, has been found convenient to use as the specific starch ester for a preferred embodiment of the invention. As indicated, the "Dry-Flo"® must be dried, to reduce its moisture content before use.

The introduction of droplets of the active material containing emulsion into the collecting powder can likewise be effected by various methods which will suggest themselves to those skilled in the art. An important concept in the practice of this step of our invention is that of keeping the particles formed in the collecting powder by the emulsion droplets separated from each other for a long enough time that they will "set up". That is, the particles should be kept separated from each other until their particulate form is permanently established, by loss of water, i.e., to such an extent that they will not agglomerate or coalesce during the most severe conditions of further processing, e.g., when the particles are spread out to dry on trays at about 45°C. The conversion of emulsion droplets to "set up" particles can obviously be attained in various ways. For example, the emulsion droplets can be let fall by a moving nozzle upon a stationary layer of collecting powder at such a space interval that the droplets do not run together. Or, the collecting powder can be presented as a moving layer (e.g., as on a conveyer belt) below a fixed nozzle adjusted to let the droplets fall at a rate such that the droplets do not run together in the powder. Still another method is represented by the spraying of emulsion droplets into an agitated mass of collecting powder, as in a tumbler or in a vessel provided with a stirrer. The particular method employed is not of the essence of the invention. However, a preferred method comprises introducing a spray of emulsion droplets into an agitated cloud or suspension in air of the particles of collecting powder. As illustrative of this latter, a method which has been found useful in quantity production comprises forcing the emulsion through a revolving spray head, having several rows of tiny orifices therein, into a suspension in air of the powdered starchy material, contained in and agitated by a revolving cylindrical drum, the drum and the spray head rotating in opposite directions so that the cloud or suspension of the starchy powder in air is swirling in a sense of rotation opposite to the entering emulsion spray. It has been found to be advantageous to include pulverized dry ice of liquid carbon dioxide in the starch bed in order to fluidize an cool the starch to less than 10°C.

The separation of the particles from the collecting powder can be accomplished by operations which are conventional per se. It has been found convenient simply to feed the mixture of powder and particles to a shaking screen of a size selected to retain the particles while passing the collecting powder. It is advantageous to so adjust the conditions under which the droplets of emulsion are formed, e.g., size of nozzle orifice, viscosity and percentage water content of emulsion, etc., as will be obvious to those skilled in the art, that the final size of the particles is substantially entirely in the range which will pass through a 10-mesh screen (opening 1,68 mm) and be retained upon a 200-mesh screen (opening 74 µ). A preferred size of particles for animal feed formulations is in the range which will pass through a 20-mesh screen (opening 841 µ) and be retained upon a 170-mesh screen (opening 88 µ). The starchy collecting powder is selected to have a smaller particle size, it being understood that for any desired size range of particles, the particles of collecting powder employed will be selected in a range of appreciably smaller size. A preferred size for collecting powder is in the range which will pass substantially entirely through a 200-mesh screen.

The particles containing the active material and formed in the collecting powder by droplets of emulsion, should be dried to a moisture content of less than 10% and preferably between about 4-6 percent. The particles can be dried by various methods. The collecting powder itself effects a certain measure of drying insofar as it absorbs and adsorbs part of the water contained in the drops of emulsion, and this drying initiates the "setting up" phenomenon (i.e., the conversion of the droplets to particles which will retain their particulate form even upon contact with other similar particles during further processing). The remaining water can be removed by various methods. For example, one can dry the entire mass, i.e., the collecting powder containing therein the particles and then separate the collecting powder from the dried particles. It is preferable to separate the particles shortly after they are formed in the powder. i.e., after their particulate form has been permanently established, but before they are completely dry, and then to dry the particles, substantially free of collecting powder, for example, by exposing them to air at room temperature, or by moderate heating in an oven at 37° to 45°C.

Heat treatment of the so obtained beadlet compositions at a temperature of from about 90°C for about 2 hours to about 180°C for less than about 1 minute, and preferably from about 105°C for 60 minutes to 150°C for 10 minutes, results in crosslinking of the gelatin sugar matrix. Assays of beadlets prepared by the method of this invention indicate no loss of e.g. vitamin A on processing; in fact, potency increased in proportion to the loss of residual beadlet moisture. The reaction involves the carbonyl of the sugar molecule with the free amino moieties of the gelatin molecule. Beadlets crosslinked by this technique are insoluble in boiling water and possess better stability to the conditions utilized in pelletizing the beadlets for animal feed compositions.

Beadlets crosslinked by this technique are insoluble in boiling water and exhibit the properties of an enteric coating; specifically, one with little or no dissolution in gastric fluid (pH^{∼}1) but completely soluble in intestinal fluid (pH^{∼}8). These characteristics would benefit any substance (vitamin, drug, carotenoid, etc.) which is degraded by the acid environment of the stomach and/or is better absorbed in the intestinal tract. The net effect of such a modification is improved bioavailability in the target species.

An important advantage of this crosslinking approach is that the beadlets maintain their initial dissolution profile with storage. Aldehyde (e.g., formaldehyde, glyceraldehyde) or ketone crosslinks tend to continue polymerizing with time, resulting in decreased bioavailability. Simulated gastric and intestinal fluids were used to determine in vitro dissolution of these preparations. Selected formulations were evaluated in preliminary bioavailability studies by the chick liver storage method using a single dose level (10,000 lU/Kg feed). The results shown below indicate that the heat hardened insoluble beadlets have excellent bioavailability when compared to soluble beadlets of the same composition (taken as 100%).

| | Avg. % Deposition | Relative Deposition % |
|---|---|---|
| Example 2 | 39.8 | 107 |
| Example 4 | 40.3 | 108 |
| Example 7B | 42.7 | 115 |

The powder compositions of the present invention comprise 20-30%, preferably 23-28%, of active material, preferably vitamin A active material, 2-20%, preferably 5-12%, of a reducing sugar; 35-45%, preferably 36-40% of gelatin; 5-20%, preferably 10-15%, of hydrophobic starch; 5-13%, preferably 6-10%, of an antioxidant; 0-15%, preferably 5-10%, of a humectant. The ranges of ingredients are in percent by weight.

The process of this invention is further illustrated by reference to the following Examples.

### Example 1

246 g of gelatin, approximately 200 Bloom, were dissolved in 300 g of distilled water by heating to about 65°C with stirring. 45 g of High Fructose Corn Syrup (55%) and 36 g of glycerin were added to the gelatin solution with stirring. 150 g of crystalline vitamin A acetate (2.9 million lU/g vitamin A activity), previously melted with 43 g of ethoxyquin (EMQ) at a temperature of about 65°C, were slowly added to the gelatin/sugar solution and homogenized until well dispersed. An additional 275 to 300 ml distilled water, previously heated to about 55°C, were added to the emulsion while stirring to provide a viscosity suitable for spraying. The emulsion was loaded into an apparatus provided with a revolving spray head and a counter-rotating drum as previously described. The drum was charged with approximately 7 kg "Dry Flo"®, previously dried to a moisture content of 3-5%, and 3.5 kg pulverized dry ice. The emulsion was sprayed into the "Dry Flo"® bed and the mixture of starch and vitamin beadlets allowed to stand overnight before being screened through a 170-mesh screen. The beadlets retained upon the screen were collected, spread out on drying trays and dried in an oven at 37°C for 7 hours to a moisture content of 4%. The beadlets were crosslinked by heating for 10 minutes on pre-heated stainless steel trays in an electric oven set to a temperature of 150°C. The beadlets thus obtained were insoluble in water, had a particle size of 20-170 mesh, a moisture content of 1% and assayed 687,000 lU vitamin A activity/g.

### Example 2

In the manner described in Example 1, an emulsion was prepared from the following ingredients:

| | | g |
|---|---|---|
| Vitamin A acetate, crystalline, 2.9 million lU/g | | 156 |
| EMQ | | 57 |
| Lactose | | 42 |
| Glycerin | | 42 |
| Low Bloom gelatin (Type A, apx. 100 Bloom) | | 240 |
| Distilled Water: | for Emulsion | 240 |
| | for Spraying | q.s. |

The emulsion thus obtained was formed into particles by means of the same apparatus and in the same manner as described in Example 1.

The beadlets were crosslinked by heating for 15 minutes at 150°C. The beadlets thus obtained have an average size substantially as in Example 1 and assayed 674,000 1U/g.

### Example 3

In the manner described in Example 1, an emulsion was prepared from the following ingredients:

| | g |
|---|---|
| Vitamin A acetate, crystalline, 2.9 million lU/g | 168 |
| BHT | 60 |
| Invert sugar | 51 |
| Glycerin | 39 |
| Low Bloom gelatin (Type B, apx. 75 Bloom) | 240 |
| Distilled water (for emulsion) | 240 |

The emulsion thus obtained was formed into particles by means of the same apparatus and in the same manner as described in Example 1.

The beadlets were crosslinked by heating for 10 minutes at 150°C. The beadlets thus obtained have an average size substantially as in Example 1 and assayed 690,000 1U/g.

### Example 4

In the manner described in Example 1, an emulsion was prepared from the following ingredients:

| | g |
|---|---|
| Vitamin A acetate, crystalline, 2.9 million lU/g | 156 |
| EMQ | 57 |
| Glucose | 42 |
| Glycerin | 42 |
| Low Bloom gelatin (Type A, apx. 100 Bloom) | 240 |
| Distilled water (for emulsion) | 240 |

The emulsion thus obtained was formed into particles by means of the same apparatus and in the same manner as described in Example 1.

The beadlets were crosslinked by heating for 30 minutes at 135°C. The beadlets thus obtained have an average size substantially as in Example 1 and assayed 699,000 1U/g.

### Example 5

In the manner described in Example 1, an emulsion was prepared from the following ingredients:

| | g |
|---|---|
| Vitamin A acetate, crystalline, 2.9 million lU/g | 156 |
| EMQ | 57 |
| Crystalline Fructose | 54 |
| Glycerin | 54 |
| Low Bloom gelatin (Type A, apx. 100 Bloom) | 216 |
| Distilled water (for emulsion) | 220 |

Adjusted emulsion to pH 8.2 with 20% w/w sodium hydroxide solution.

The emulsion thus obtained was formed into particles by means of the same apparatus and in the same manner as described in Example 1.

The beadlets were crosslinked by heating for 60 minutes at 105°C. The beadlets thus obtained have an average size substantially as in Example 1 and assayed 686,000 1U/g.

### Example 6

In the manner described in Example 1, an emulsion was prepared from the following ingredients:

| | g |
|---|---|
| Vitamin A acetate, crystalline, 2.9 million lU/gm | 150 |
| EMQ | 43 |
| Fructose Corn Syrup (55%) | 92 |
| Gelatin (apx. 200 Bloom) | 246 |
| Distilled water (for emulsion) | 300 |

The emulsion thus obtained was formed into particles by means of the same apparatus and in the same manner as described in Example 1.

The beadlets were crosslinked by heating for 10 minutes at 150°C. The beadlets thus obtained have an average size substantially as in Example 1 and assayed 684,000 1U/g.

### Example 7

In the manner described in Example 1. an emulsion was prepared from the following ingredients:

| | g |
|---|---|
| Vitamin A acetate, crystalline, 2.9 million lU/g | 156 |
| EMQ | 57 |
| Invert sugar | 55 |
| Glycerin | 42 |
| Low Bloom gelatin (Type A, apx. 100 Bloom) | 240 |
| Distilled water (for emulsion) | 240 |

The emulsion thus obtained was formed into particles by means of the same apparatus and in the same manner as described in Example 1.

The beadlets were divided into three portions, and the portions were crosslinked using the following conditions:

| | Heat Treatment | Assay (lU/g) |
|---|---|---|
| 7A | 150°C for 10 minutes | 712,000 |
| 7B | 135°C for 25 minutes | 690,000 |
| 7C | 120°C for 50 minutes | 712,000 |

### Example 8

In the manner described in Example 1, an emulsion was prepared from the following ingredients:

| | g |
|---|---|
| Vitamin A acetate, crystalline, 2.9 million lU/g | 156 |
| EMQ | 57 |
| Invert sugar | 55 |
| Glycerin | 42 |
| Hydrolyzed Gelatin | 270 |
| Distilled water (for emulsion) | 270 |

The emulsion thus obtained was formed into particles by means of the same apparatus and in the same manner as described in Example 1.

The beadlets were crosslinked by heating for 10 minutes at 150°C. The beadlets thus obtained have an average size substantially as in Example 1 and assayed 701,000 1U/g.

### Example 9

In the manner described in Example 1, an emulsion was prepared from the following ingredients:

| | g |
|---|---|
| Vitamin A acetate, crystalline, 2.9 million lU/gm | 150 |
| EMQ | 43 |
| High Fructose Corn Syrup (55%) | 45 |
| Propylene Glycol | 36 |
| Gelatin (apx. 200 Bloom) | 246 |
| Distilled water (for emulsion) | 300 |

The emulsion thus obtained was formed into particles by means of the same apparatus and in the same manner as described in Example 1.

The beadlets were crosslinked by heating for 10 minutes at 150°C. The beadlets thus obtained have an average size substantially as in Example 1 and assayed 694,000 1U/g.

## Claims

1. Process for the manufacture of preparations in beadlet form containing one or more fat-soluble vitamins and/or one or more carotenoids, which comprises the steps of forming an emulsion containing the active material, water, gelatin and a sugar which has a carbonyl group capable of reacting with the free aminogroup of the gelatin molecule, converting the emulsion to droplets, collecting the droplets in a mass of starchy powder in such a manner that the droplets are kept separated from each other until their particulate form is permanently established and separating the obtained particles from the starchy collecting powder, and is characterized in that these particles are heat treated at a temperature of from about 90°C to about 180°C.

2. Process according to claim 1, characterized in that the heat treatment is carried out at a temperature from about 90°C for two hours to about 180°C for less than about a minute.

3. Process according to claim 1 or 2, characterized in that the heat treatment is carried out at from about 105°C for 60 minutes to about 150°C for 10 minutes.

4. Process according to any one of claims 1 to 3, characterized in that preparations containing a fat soluble vitamin active material are prepared.

5. A preparation in beadlet form containing one or more fat-soluble vitamins and/or one or more carotenoids, prepared according to the process as claimed in any one of claims 1 to 3.

6. A preparation according to claim 5, comprising 20-30% of active material, 2-20% of a reducing sugar, 35-45% of a gelatin, 5-20% of a hydrophobic starch, 5-13% of an antioxidant and 0-15% of a humectant.

7. A preparation according to claims 5 or 6, wherein the active material is a fat soluble vitamin active material.

## Patentansprüche

1. Verfahren zur Herstellung von kugelförmigen Präparaten, welche ein oder mehrere fettlösliche Vitamine und/oder ein oder mehrere Carotinoide enthalten, und das als Schritte die Bildung einer Emulsion, welche das aktive Material, Wasser, Gelatine und einen Zucker enthält, der eine Carbonylgruppe aufweist, die mit der freien Aminogruppe des Gelatinemoleküls reagieren kann, die Umwandlung der Emulsion in Tröpfchen, das Sammeln der Tröpfchen in einer Masse aus Stärkepulver in einer Art, daß die Tröpfchen voneinander getrennt bleiben bis sich ihre besondere Form dauerhaft ausgebildet hat, und die Trennung der so erhaltenen Partikel von dem Stärkepulver umfaßt, dadurch gekennzeichnet, daß diese Partikel einer Wärmebehandlung bei einer Temperatur von etwa 90°C bis etwa 180°C unterzogen werden.

2. Verfahren nach Anspruch 1, dadurch gekennzeichnet, daß die Wärmebehandlung bei einer Temperatur von etwa 90°C während 2 Stunden bis etwa 180°C während weniger als etwa eine Minute durchgeführt wird.

3. Verfahren nach Anspruch 1 oder 2, dadurch gekennzeichnet, daß die Wärmebehandlung durchgeführt wird von etwa 105°C während 60 Minuten bis etwa 150°C während 10 Minuten.

4. Verfahren nach einem der Ansprüche 1 bis 3, dadurch gekennzeichnet, daß Präparate hergestellt werden, welche ein fettlösliches Vitamin-aktives Material enthalten.

5. Ein kugelförmiges Präparat welches ein oder mehrere fettlösliche Vitamine und/oder ein oder mehrere Carotinoide enthält, hergestellt nach einem Verfahren gemäß einem der Ansprüche 1 bis 3.

6. Ein Präparat gemäß Anspruch 5, welches 20-30% des aktiven Materials, 2-20% eines reduzierenden Zuckers, 35-45% Gelatine, 5-20% einer hydrophoben Stärke, 5-13% eines Antioxidans und 0-15% eines Feuchthaltemittels enthält.

7. Ein Präparat gemäß Anspruch 5 oder 6, in welchem das aktive Material ein fettlösliches Vitamin-aktives Material ist.

## Revendications

1. Procédé pour la fabrication de préparations sous la forme de petites perles contenant une ou plusieurs vitamines solubles dans les graisses et/ou un ou plusieurs caroténoïdes, qui comprend les étapes de formation d'une émulsion contenant le matériau actif, de l'eau, de la gélatine et un sucre, qui comprend un groupe carbonyle capable de réagir avec le groupe amino libre de la molécule gélatine, de transformation de l'émulsion en gouttelettes, de collecte des gouttelettes dans une masse de poudre amylacée d'une manière telle que les gouttelettes sont maintenues dans un état séparé les unes des autres jusqu'à ce que leur forme particulaire soit étsblie de manière permanente et de séparation des particules obtenues hors de la poudre amylacée de collecte, et qui est caractérisé en ce que l'on traite thermiquement ces particules à une température comprise entra environ 90°C et environ 180°C.

2. Procédé selon la revendication 1, caractérisé en ce que l'on effectue le traitement thermique à une température comprise entre environ 90°C pendant deux heures et environ 180°C pendant moins d'environ une minute.

3. Procédé selon la revendication 1 ou 2, caractérisé en ce que l'on effectue le traitement thermique entre environ 105°C pendant 60 minutes et environ 150°C pendant 10 minutes.

4. Procédé selon l'une quelconque des revendications 1 à 3, caractérisé en ce que l'on prépare des préparations contenant un matériau actif de vitamine soluble dans les graisses .

5. Préparation sous la forme de petites perles contenant une ou plusieurs vitamines solubles dans les graisses et/ou un ou plusieurs caroténoïdes, préparée conformément au procédé salon l'une quelconque des revendications 1 à 3.

6. préparation selon la revendication 5, qui comprend 20-30% du matériau actif, 2-20% d'un sucre réducteur, 35-45% d'une gélatine, 5-20% d'un amidon hydrophobe, 5-13% d'un antioxydant et 0-15% d'un humectant

7. Préparation selon la revendication 5 ou 6, dans laquelle le matériau actif est un matériau actif de vitamine soluble dans les graisses.
